# EUROPEAN PATENT APPLICATION

(11) **EP 4 541 274 A1**
(43) Date of publication of application: **23.04.2025**
(21) Application number: 22953215.5
(22) Date of filing: 23.12.2022
(51) Int. Cl.: A61B 5/11, A61B 5/107

(54) **INFORMATION PROCESSING DEVICE, INFORMATION PROCESSING SYSTEM, INFORMATION PROCESSING METHOD, AND PROGRAM**

(30) Priority: 28.07.2022 JP 2022120403
(71) Applicant: Suntory Holdings Limited, Osaka 530-8203 (JP)
(72) Inventor: YAMAMURA, Junki, Kyoto 6190284 (JP); TAKAGI, Motoshige, Osaka-shi Osaka 530-8204 (JP); NAKAGAWA, Kaname, Osaka-shi Osaka 530-8204 (JP)
(74) Representative: Vossius & Partner Patentanwälte Rechtsanwälte mbB
(86) International application number: PCT/JP2022/047668
(87) International publication number: WO 2024/024127

(57) **Abstract**

In order to address the problem that specifying information and giving advice on a kinesiological state of the body of a user require specialized knowledge and experience and the effort and cost required are relatively high, an information processing apparatus 100 includes: a sole information acquiring unit 141 that acquires sole information on a sole of a user; a determination information acquiring unit 149 that acquires determination information on a kinesiological state of the body of the user, using the sole information and learning information that is prepared in advance; an output information acquiring unit 151 that acquires output information based on the determination information; and an output unit 161 that outputs the output information. Accordingly, it is possible to easily specify information on a kinesiological state of the body of a user.

## Description

### Technical Field

The present invention relates to an information processing apparatus, an information processing system, an information processing method, and a program that perform information processing using information on a sole of a user.

### Background Art

Conventionally, there are apparatuses configured to output health-related information to users (see Patent Document 1 below, for example).

Furthermore, Patent Document 2 below describes obtaining symptom data concerning physical symptoms and physical conditions of a subject based on skeletal data generated based on data of captured images or video of the body of the subject.

### Citation List

### Patent Documents

Patent Document 1: JP 2021-135554A
Patent Document 2: JP 2021-115471A

### Summary of Invention

### Technical Problem

In recent years, for example, it is sometimes performed to specify information on the condition of an individual user such as his or her posture or way of walking, and to provide the specified information to the user or to give various kinds of advice to the user based on the specified information. In general, specifying information and giving advice on a kinesiological state of the body of a user in this manner require specialized knowledge and experience, and the effort and cost required are relatively high.

In relation to these issues, according to the technique disclosed in Patent Document 2 above, it is possible to obtain symptom data of a subject through information processing. However, such a technique requires the generation of skeletal data, which is effort-consuming and involves a heavy burden of information processing.

The present invention has been made in order to address the above-described issues, and it is an object thereof to provide an information processing apparatus, an information processing system, an information processing method, and a program that can easily specify information on a kinesiological state of the body of a user.

### Solution to Problem

A first aspect of the present invention is directed to an information processing apparatus including: a sole information acquiring unit that acquires sole information on a sole of a user; a determination information acquiring unit that acquires determination information on a kinesiological state of the body of the user, using the sole information and learning information that is prepared in advance; and an output unit that outputs output information that is based on the determination information.

With this configuration, it is possible to easily specify information on a kinesiological state of the body of a user.

Furthermore, a second aspect of the present invention is directed to the information processing apparatus according to the first aspect, wherein the sole information contains information on a ground contacting state in which the user's sole is in contact with a ground surface.

With this configuration, it is possible to specify information on a kinesiological state of the body of a user, based on a ground contacting state of a sole.

Furthermore, a third aspect of the present invention is directed to the information processing apparatus according to the second aspect, wherein the sole information contains information indicating the ground contacting state of the user's sole in chronological order.

With this configuration, it is possible to specify information on a kinesiological state of the body of a user, based on a change in the ground contacting state of a sole.

Furthermore, a fourth aspect of the present invention is directed to the information processing apparatus according to any one of the first to third aspects, wherein the sole information contains a captured image of the user's sole.

With this configuration, it is possible to specify information on a kinesiological state of the body of a user, based on easily obtainable information.

Furthermore, a fifth aspect of the present invention is directed to the information processing apparatus according to any one of the first to fourth aspects, wherein the sole information contains information on a pressure distribution of the user's sole that is in contact with a ground surface.

With this configuration, it is possible to specify information on a kinesiological state of the body of a user, based on a ground contacting state of a sole.

Furthermore, a sixth aspect of the present invention is directed to the information processing apparatus according to any one of the first to fifth aspects, wherein the sole information contains information indicating a three-dimensional shape of the user's sole.

With this configuration, it is possible to specify information on a kinesiological state of the body of a user, based on easily obtainable information.

Furthermore, a seventh aspect of the present invention is directed to the information processing apparatus according to any one of the first to sixth aspects, wherein the determination information acquiring unit is configured to perform processing for increasing resolution of the sole information that is an image, and use a processing result thereof and the learning information to acquire the determination information.

With this configuration, it is possible to accurately specify information on a kinesiological state of the body of a user.

Furthermore, an eighth aspect of the present invention is directed to the information processing apparatus according to any one of the first to seventh aspects, wherein the sole information contains shape information on a shape of the user's sole.

With this configuration, it is possible to accurately specify information on a kinesiological state of the body of a user.

Furthermore, a ninth aspect of the present invention is directed to the information processing apparatus according to the eighth aspect, wherein the shape information contains at least one of a sole length and a sole width of the user.

With this configuration, it is possible to accurately specify information on a kinesiological state of the body of a user.

Furthermore, a tenth aspect of the present invention is directed to the information processing apparatus according to any one of the first to ninth aspects, further including: a user information acquiring unit that acquires information that is on the user and is different from the sole information, as user information, wherein the determination information acquiring unit acquires the determination information using the user information.

With this configuration, it is possible to accurately specify information on a kinesiological state of the body of a user.

Furthermore, an eleventh aspect of the present invention is directed to the information processing apparatus according to the tenth aspect, wherein the determination information acquiring unit acquires the determination information, using learning information corresponding to the user information among multiple pieces of learning information that are prepared in advance.

With this configuration, it is possible to accurately specify information on a kinesiological state of the body of a user.

Furthermore, a twelfth aspect of the present invention is directed to the information processing apparatus according to any one of the first to eleventh aspects, wherein the determination information contains information on each of two or more findings regarding contact of a human sole with the ground.

With this configuration, it is possible to easily specify information on each of two or more findings regarding contact of a user's sole with the ground.

Furthermore, a thirteenth aspect of the present invention is directed to the information processing apparatus according to the twelfth aspect, wherein the determination information contains respective scores of the two or more findings.

With this configuration, it is possible to easily specify respective scores of two or more findings regarding contact of a user's sole with the ground.

Furthermore, a fourteenth aspect of the present invention is directed to the information processing apparatus according to the twelfth or thirteenth aspect, wherein the determination information acquiring unit acquires information on each of the two or more findings, using learning information corresponding to the findings among multiple pieces of learning information that are prepared in advance.

With this configuration, it is possible to accurately specify information on each of two or more findings regarding contact of a user's sole with the ground.

Furthermore, a fifteenth aspect of the present invention is directed to the information processing apparatus according to any one of the first to fourteenth aspects, wherein the output information contains a determination result regarding a walking posture of the user.

With this configuration, it is possible to easily output a determination result regarding a walking posture of a user.

Furthermore, a sixteenth aspect of the present invention is directed to the information processing apparatus according to any one of the first to fifteenth aspects, wherein the output information contains a suggestion for improving the kinesiological state of the body of the user.

With this configuration, it is possible to easily output a suggestion for improving the kinesiological state of the body of a user.

Furthermore, a seventeenth aspect of the present invention is directed to the information processing apparatus according to any one of the first to sixteenth aspects, wherein the output information contains information on a product recommended for the user to ingest.

With this configuration, it is possible to easily output information on a product recommend for a user to ingest.

Furthermore, an eighteenth aspect of the present invention is directed to the information processing apparatus according to any one of the first to seventeenth aspects, wherein the output information contains information on a symptom that may be currently occurring in the user.

With this configuration, it is possible to easily output information on a symptom that may be currently occurring in a user.

Furthermore, a nineteenth aspect of the present invention is directed to the information processing apparatus according to any one of the first to eighteenth aspects, wherein the output information contains information on a risk of a symptom that occurs in the user in the future.

With this configuration, it is possible to easily output information on a risk of a symptom that occurs in a user in the future.

Furthermore, a twentieth aspect of the present invention is directed to the information processing apparatus according to the nineteenth aspect, wherein the output information contains two or more pieces of information on risks of symptoms that occur, and the output unit outputs the two or more pieces of information on risks of symptoms that occur, in an order corresponding to when the respective symptoms are expected to occur.

With this configuration, it is possible to easily ascertain the risks of two or more symptoms, along with when they are expected to occur.

Furthermore, a twenty-first aspect of the present invention is directed to the information processing apparatus according to any one of the first to twentieth aspects, wherein the output information is information acquired based on determination information acquired for the user this time and determination information acquired for the same user in the past.

With this configuration, it is possible to output the output information that is based on the respective determination information of the past and the present.

Furthermore, a twenty-second aspect of the present invention is directed to an information processing system including: an information processing apparatus according to any one of the first to twenty-first aspects; and an information acquiring apparatus capable of communicating with the information processing apparatus, wherein the information acquiring apparatus is configured to record information on a sole of a user and output a recorded result as sole information, and the sole information acquiring unit is configured to acquire the sole information output by the information acquiring apparatus.

With this configuration, it is possible to easily specify information on a kinesiological state of a user.

### Advantageous Effects of Invention

According to the information processing apparatus and the like of the present invention, it is possible to easily specify information on a kinesiological state of the body of a user.

### Brief Description of Drawings

FIG. 1 is a diagram showing an overview of an information processing system according to an embodiment of the present invention.
FIG. 2 is a block diagram of the information processing system in the embodiment.
FIG. 3 is a flowchart showing an example of an operation of an information processing apparatus in the embodiment.
FIG. 4 is a flowchart showing an example of determination processing by the information processing apparatus in the embodiment.
FIG. 5 is a flowchart showing an example of output information acquisition processing by the information processing apparatus in the embodiment.
FIG. 6 is a flowchart showing a modified example of the output information acquisition processing by the information processing apparatus in the embodiment.
FIG. 7 is a flowchart showing a modified example of the determination processing by the information processing apparatus in the embodiment.
FIG. 8 is a diagram showing a specific example of a display screen of output information of the information acquiring apparatus in the embodiment.
FIG. 9 is a diagram showing an example of information indicating the correspondence between determination information and output information or the like according to a modified example of this embodiment.
FIG. 10 is a flowchart showing an example of output information acquisition processing by the information processing apparatus according to a modified example of this embodiment.
FIG. 11 is a diagram showing a specific example of a display screen of output information of the information acquiring apparatus according to a modified example of this embodiment.
FIG. 12 is a schematic view of a computer system in the foregoing embodiment.
FIG. 13 is a block diagram of the computer system in the embodiment.

### Description of Embodiment

Hereinafter, an embodiment of an information processing apparatus and the like will be described with reference to the drawings. It should be noted that constituent elements denoted by the same reference numerals in the embodiments perform similar operations, and thus a description thereof may not be repeated.

The terms used below are generally defined as follows. The meanings of these terms do not always have to be construed as indicated herein, and have to be construed in light of individual explanations below, if any, for example.

The term "user" refers to a user who uses an information processing system. Users may include users who perform an operation or the like on an information processing system (which may be referred to as operators, administrators, etc.), as well as users from which determination information is to be acquired by the information processing system (which may be referred to as examinees, subjects, etc.). An operator and an examinee may be the same person or may be different people.

The term "identifier for a matter" is a letter, a symbol, or the like for uniquely identifying the matter. The identifier is an ID, for example, but may be any type of information with which the corresponding matter can be identified. That is to say, the identifier may be the name of the matter itself that it indicates, or symbols that are combined so as to uniquely correspond to the matter.

The term "acquiring" may include acquiring a matter that is input by a user or the like, or acquiring information stored in the apparatus or another apparatus (the information may be information stored in advance or information generated through information processing in the apparatus). The acquiring information stored in another apparatus may include acquiring information stored in the other apparatus via an API or the like, or acquiring the content of a document file provided by the other apparatus (the content includes the webpage content, etc.). The acquiring may include acquiring information in a different format based on the original information, such as acquiring information through optical character reading of an image file.

Furthermore, a so-called machine learning method may be used to acquire information. A machine learning method may be used as follows, for example. That is to say, learning information in which a particular type of input information is taken as input and the type of information that is to be acquired is taken as output is configured using a machine learning method. For example, two or more pairs of input information and acquired information that is taken as output (which may be referred to as output information) are prepared in advance, the two or more pairs of information are given to a module for configuring learning information of machine learning to configure learning information, and the configured learning information is accumulated in a storage unit. The learning information may also be said to be a learning model or a classifier. There is no limitation on the machine learning method, and examples thereof include deep learning, random forests, SVM, and the like. For example, functions in various machine learning frameworks and various existing libraries, such as fastText, tinySVM, random forest, and TensorFlow, can be used for the machine learning.

The term "learning information" is not limited to those obtained through machine learning. The learning information may be a table indicating the correspondence between an input vector that is based on input information or the like and acquired information, for example. In this case, acquired information corresponding to a feature vector that is based on input information may be acquired from the table, or a vector that is close to a feature vector that is based on input information may be generated using two or more input vectors in the table and parameters for weighting the input vectors, and acquired information corresponding to the input vectors and the parameters used for generation may be used to acquire final acquired information. Such acquisition of information using learning information may be referred to as acquisition using correspondence. The learning information may be a function or the like that represents the relationship between an input vector that is based on input information or the like and information for generating acquired information, for example. In this case, for example, information corresponding to a feature vector that is based on input information may be obtained using a function, and the obtained information may be used to acquire acquired information. Such acquisition of information using learning information may be referred to as acquisition using a function.

The term "to output information" is a concept that encompasses display on a display screen, projection using a projector, printing by a printer, output of a sound, transmission to an external apparatus, accumulation in a recording medium, and delivery of a processing result to another processing apparatus or another program. Specifically, for example, this concept encompasses enabling information to be displayed on a webpage, transmission as an email or the like, and outputting information for printing.

The term "to accept information" is a concept that encompasses accepting information input from an input device such as a keyboard, a mouse, or a touch screen, receiving information transmitted from another apparatus or the like via a wired or wireless communication line, and accepting information read from a recording medium such as an optical disk, a magnetic disk, or a semiconductor memory.

The term "to update various types of information stored in an information processing apparatus or the like" is a concept that encompasses changing the stored information, adding new information to the stored information, and deleting part or the whole of the stored information.

### Embodiment

In this embodiment, the information processing apparatus acquires a determination result using sole information on a sole of a user and learning information, and outputs the output information based on the determination result. The sole information is, for example, information on a state in which a sole is in contact with the ground, information indicating the ground contacting state in chronological order, or the like. The sole information may be information on the pressure distribution of a sole that is in contact with a ground surface or the like. The sole information is not limited to these, and may be, for example, a captured image of a sole or information indicating a three-dimensional shape of a sole, for example. The information processing apparatus may be configured to acquire a determination result, using information obtained by increasing the resolution of sole information that is an image using a generative adversarial network (GAN) method or the like, and learning information. The information processing apparatus may acquire shape information from sole information and acquire a determination result using the sole information / the shape information and learning information, or may acquire a determination result using user information of a user and learning information. The determination result is, for example, information such as respective scores of two or more findings. The output information may contain, for example, a determination result regarding a walking posture of the user, or may contain a suggestion for improving a walking posture. The output information may contain information on a product recommended for a user to ingest. Hereinafter, the configuration of an information processing system including the thus configured information processing apparatus will be described.

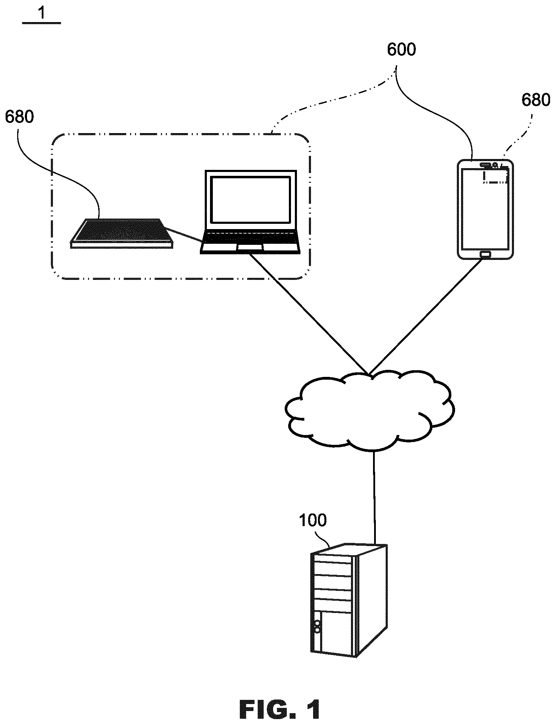

FIG. 1 is a diagram showing an overview of an information processing system 1 according to an embodiment of the present invention.

In this embodiment, the information processing system 1 includes an information processing apparatus 100 and information acquiring apparatuses 600. The information processing apparatus 100 and the information acquiring apparatuses 600 can communicate with each other via a network such as a local area network or the Internet, for example. The configuration of the information processing system 1 is not limited to this. Any number of respective apparatuses may be included in the information processing system 1, and other apparatuses may be included in the information processing system 1.

Users of the information processing system 1 can use the information processing system 1 by using the information acquiring apparatuses 600. In the drawing, for example, a portable information terminal device such as a so-called smart phone and a personal computer (PC) such as a laptop computer are shown as the information acquiring apparatuses 600, but they are merely an example, and, for example, tablet-type information terminal devices, measuring devices that are not generally called portable information terminal devices or PCs, and the like may also be used as the information acquiring apparatuses 600. The information acquiring apparatuses 600 may be referred to as terminal devices.

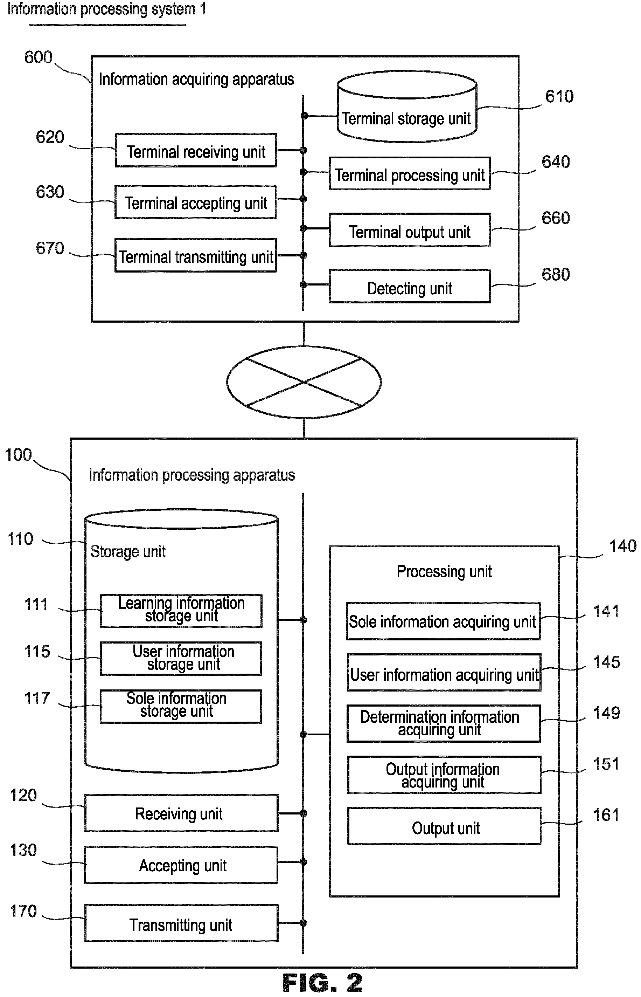

FIG. 2 is a block diagram of the information processing system 1 in this embodiment.

As shown in the drawing, the information processing apparatus 100 includes a storage unit 110, a receiving unit 120, an accepting unit 130, a processing unit 140, and a transmitting unit 170. The information processing apparatus 100 is, for example, a server apparatus.

The storage unit 110 includes a learning information storage unit 111, a user information storage unit 115, and a sole information storage unit 117.

Learning information acquired in advance is stored in the learning information storage unit 111. The learning information may be referred to as a learning model, a classifier, a predictive model, or a learned model. The learning information is generated using so-called machine learning methods. The learning information is generated in the information processing apparatus 100, for example, as will be described later, and stored in the learning information storage unit 111, but there is no limitation to this. That is to say, learning information generated in an apparatus different from the information processing apparatus 100 may be stored in the learning information storage unit 111.

In this embodiment, the learning information is generated in such a way that information containing the sole information is taken as information that is input and determination information is taken as information that is output. In other words, the learning information can be said to be information that is used to output determination information by being applied to input information containing sole information.

The sole information is information on a sole of a user. In this embodiment, the sole information is information that is based on a captured image, captured by the camera, of a user's sole that is in contact with a ground surface. That is to say, in this embodiment, the sole information contains information on a ground contacting state in which the user's sole is in contact with a ground surface. The information that is based on a captured image may be the captured image itself or information acquired using the captured image. The information acquired using a captured image may be, for example, an image obtained as a result of performing predetermined image processing or information acquired as a result of performing predetermined image recognition. The information on a ground contacting state may be a captured image or an image obtained by processing the captured image, or may be those containing a value of a predetermined indicator concerning a user's sole that is in contact with a ground surface. The predetermined indicator may be, for example, an indicator concerning the shape such as the length of a sole that is in contact with the ground (sole length) or the width of a sole that is in contact with the ground (sole width), or may be, for example, other indicators such as the ground contacting area of the sole and the position of the center of gravity of the ground contacting portion.

The sole information may be information indicating a change in the ground contacting state of a user's sole in chronological order. For example, it may be a moving image (an example of a captured image) of a user's sole that is in contact with a ground surface. It may be two or more images, captured at intervals, of a user's sole that is in contact with a ground surface. For example, it may be those containing values of a predetermined indicator as described above in chronological order. For example, the sole information may be, for example, information indicating, in chronological order, a change in the ground contacting state of a user's sole in a predetermined motion state such as walking or jumping.

The determination information in this embodiment is information on a kinesiological state of the body of a user. The kinesiological state is, for example, a walking posture, a standing posture, a way of walking, or the like. The kinesiological state may be the state of the musculoskeletal system such as the state of the skeleton or the state of the muscles. The determination information is whether or not each finding regarding a kinesiological state is matched or respective scores of multiple findings. The determination information may also be said to be diagnostic information.

In this embodiment, the determination information contains, for example, information on predetermined findings regarding contact of a human sole with the ground. More specifically, for example, it is information on each of two or more findings that are taken into account in distinguishing whether or not a user has overpronation in his or her way of walking (walking posture). These multiple findings refer to four findings consisting of: a user has a floating toe (floating toe); the ground contacting state of the base of toes is not good (base contact); a user has an arch that collapses flat (flat foot); and a user has a high arch foot (high arch), but there is no limitation to these. The finding of floating toe may be that a user does not have a floating toe, the finding of base contact may be that the ground contacting state is good, the finding of flat foot may be that a user does not have a flat foot, and the finding of high arch may be that a user does not have a high arch. In this embodiment, the determination information contains, for example, respective scores of the four findings consisting of floating toe, base contact, flat foot, and high arch. The determination information may contain information on findings regarding knee osteoarthritis, low back pain (hernia), or the like.

The learning information may be information such as a correspondence table prepared to enable the above-described acquisition using correspondence. The correspondence table may be, for example, any information representing the correspondence between the vector of input information and the determination information. The learning information may be information on a function prepared to enable the above-described acquisition using a function.

In this embodiment, multiple pieces of learning information are prepared. The learning information is associated with each of the four findings of the determination information and stored in the learning information storage unit 111. That is to say, learning information corresponding to output of a floating toe score, learning information corresponding to output of a base contact score, learning information corresponding to output of a flat foot score, and learning information corresponding to output of a high arch score are stored in the learning information storage unit 111. The corresponding learning information is used for the output of each finding score.

User information on a user who is an examinee is stored in the user information storage unit 115. In this embodiment, the user information is information in which a user identifier for identifying a user is associated with information on the user. The user information may contain various types of information. For example, it may contain information transmitted from the information acquiring apparatuses 600, information on a user acquired by the information processing apparatus 100 as will be described later, and the like. The information transmitted from the information acquiring apparatuses 600 may contain various attribute values (e.g., sex, age, weight, height, presence or absence of certain diseases, etc.) of a user. The information on a user acquired by the information processing apparatus 100 corresponds to, for example, determination information, later-described output information, and the like. User information transmitted from other external apparatuses and the like may be stored in the user information storage unit 115.

In the description of this embodiment, the sole information among the information on a user is treated as not included in the user information. However, the sole information may be considered to be included in the user information. In that case, the sole information storage unit 117 can be considered to be included in the user information storage unit 115.

Sole information is stored in the sole information storage unit 117. In this embodiment, the sole information is, for example, information acquired and transmitted by the information acquiring apparatuses 600, but there is no limitation to this. For example, sole information acquired in the information processing apparatus 100 based on an image or the like of a sole may be stored in the sole information storage unit 117. In the sole information storage unit 117, sole information of each user is stored in association with a user identifier of that user.

The receiving unit 120 receives information transmitted from other apparatuses. The receiving unit 120 stores the received information, for example, in the storage unit 110. In this embodiment, for example, a user who is an operator or a user who is an examinee performs input or the like of information using the information acquiring apparatus 600, and transmits it to the information processing apparatus 100. In this case, the information is transmitted in association with a user identifier of the user who is an examinee. The receiving unit 120 can store each pieces of transmitted information in the storage unit 110 in association with the user identifier. As will be described later, in this embodiment, sole information transmitted from each of the information acquiring apparatuses 600 can be received and stored in the storage unit 110 in association with a user identifier.

The accepting unit 130 accepts information input using an input part (not shown) connected to the information processing apparatus 100. The accepting unit 130 stores the accepted information, for example, in the storage unit 110. The information may be input by any input part such as a numeric keypad, a keyboard, a mouse, or a menu screen. The accepting unit 130 may accept information input through an input operation performed using a reading device (e.g., code reader, etc.) connected to the information processing apparatus 100 (e.g., including information read by the device).

The accepting unit 130 may be considered to accept information received by the receiving unit 120, as information input to the information processing apparatus 100. That is to say, the input of information to the information processing apparatus 100 may be construed to mean that the information is indirectly input to the information processing apparatus 100 by a user who is an operator via the information acquiring apparatus 600 or the like or that the information is directly input to the information processing apparatus 100 by a user using an input part. The processing in which a user executes a program for automatically generating information or gives various types of information to a program, thereby causing it to function so that information is given to the information processing apparatus 100 may be considered as the input of information to the information processing apparatus 100.

The processing unit 140 includes a sole information acquiring unit 141, a user information acquiring unit 145, a determination information acquiring unit 149, an output information acquiring unit 151, and an output unit 161. The processing unit 140 performs various types of processing. The various types of processing are, for example, processing that is performed by the units of the processing unit 140 as follows.

The sole information acquiring unit 141 acquires sole information of a user. In this embodiment, the sole information acquiring unit 141 acquires sole information transmitted from the information acquiring apparatuses 600 and received by the receiving unit 120. The sole information acquiring unit 141 can acquire sole information corresponding to a user, from the sole information storage unit 117.

The user information acquiring unit 145 acquires user information of a user. In this embodiment, the user information acquiring unit 145 can acquire user information, from the user information storage unit 115.

The determination information acquiring unit 149 acquires determination information for the user, using the sole information and learning information that is prepared in advance.

In this embodiment, the determination information acquiring unit 149 acquires determination information containing respective scores of the four findings, using the learning information stored in the learning information storage unit 111. The determination information acquiring unit 149 is configured to be able to acquire information on each of the four findings, using learning information corresponding to the respective findings. This allows for acquiring more accurate information for each of the findings.

In this embodiment, the determination information acquiring unit 149 is configured to perform processing for increasing the resolution of sole information that is a captured image, and use sole information (an image that is based on the captured image), which is a processing result thereof, and the learning information to acquire determination information. The learning information is configured using sole information having high resolution as with the sole information whose resolution has been increased. The determination information acquiring unit 149 can acquire sole information whose resolution has been increased, for example, using a known generative adversarial network (GAN) method or the like. If an image whose resolution has been increased is used in this manner, it is possible to easily acquire more accurate determination information.

Furthermore, in this embodiment, the sole information contains shape information on a shape of a user's sole. For example, the determination information acquiring unit 149 is configured to acquire shape information, and use the sole information including the shape information and the learning information to acquire determination information. The determination information acquiring unit 149 may also be said to be configured to acquire determination information, using the shape information, and the sole information excluding the shape information. The determination information acquiring unit 149 is configured to acquire determination information, for example, such that the shape information is contained in the input information. In this case, the learning information is configured such that the sole information and the shape information are taken as the input information and the determination information is taken as the output information.

In this embodiment, the shape information that is used is, for example, values of the sole width (the widest horizontal dimension) and the sole length (the length from the longest fingertip to the heel). However, there is no limitation to this, and the shape information may be either the sole width or the sole length. The shape information may be the toe length, the heel width, the first toe angle, the fifth toe angle, the hallux valgus angle, or the like.

The determination information acquiring unit 149 is configured, for example, to acquire shape information from the sole information. The determination information acquiring unit 149 is configured, for example, to acquire shape information by performing a predetermined image recognition process on sole information that is an image. That is to say, the determination information acquiring unit 149 may be configured, for example, to acquire shape information from sole information that is based on the captured image, and use the sole information that is based on the captured image (the sole information excluding the shape information) and the sole information including the shape information to acquire determination information. The determination information acquiring unit 149 may be configured to acquire information input by a user or the like, as the shape information. The shape information is not limited to the examples mentioned above, and may be, for example, information representing an external shape of the ground contacting portion of a sole.

If determination information is acquired using shape information in this manner, it is possible to acquire more accurate determination information.

The determination information acquiring unit 149 may be configured to select learning information corresponding to the shape information, and apply the selected learning information to the input information to acquire determination information. In this case as well, it can be said that determination information is acquired using the sole information including the shape information and the learning information. In this case, it is sufficient that learning information is configured and accumulated in the learning information storage unit 111 in advance for each case in which the shape information matches a predetermined condition (e.g., for each predetermined range of shape information).

Furthermore, in this embodiment, the determination information acquiring unit 149 may be configured to acquire determination information, using learning information corresponding to the user information among multiple pieces of learning information that are prepared in advance. In this case, it is sufficient that learning information is configured and accumulated in the learning information storage unit 111 in advance for each case in which an attribute value of the user information matches a predetermined condition (e.g., for each weight range, each BMI range, each age group, etc.).

If determination information is acquired using learning information corresponding to user information in this manner, it is possible to acquire more accurate determination information.

The output information acquiring unit 151 acquires output information based on the determination information. The output information is, for example, information generated based on scores, which are determination information, but may be the determination information itself.

In this embodiment, the output information acquiring unit 151 is configured to acquire output information containing information on a kinesiological state of the body of a user, and more specifically, a determination result regarding a walking posture of the user. More specifically, in this embodiment, the output information acquiring unit 151 is configured, for example, to acquire a determination result as to whether or not a user has overpronation in his or her walking posture, as the output information. That is to say, the degree at which a user can be said to have overpronation increases in the cases in which the user can be said to have a floating toe, in which the ground contacting state of the base of toes is not good, in which the user has an arch that collapses flat, and in which the user has a high arch foot. The output information acquiring unit 151 is configured, for example, to calculate a result of comparison between a score of each finding and a predetermined threshold corresponding to the finding, and determine whether or not a predetermined determination condition for overpronation is satisfied, based on the comparison results of the findings. In this case, examples of the determination condition include that the number of findings with comparison results in which the user can be said to have a tendency to overpronate is greater than or equal to a predetermined number. However, the determination method is not limited to this. For example, it is also possible to make a determination (which may be referred to as diagnosis) as to having overpronation, by determining whether or not a predetermined determination condition is satisfied by a result of comparison between the total score of the findings and a predetermined threshold value. For example, the output information acquiring unit 151 may acquire a determination result using learning information (hereinafter, also referred to as second learning information for convenience) configured in advance such that information containing scores of two or more findings is taken as information that is input and a determination result as to whether or not a user has overpronation is taken as information that is output. In this case as well, any of the following methods may also be used: acquisition by a machine learning method; acquisition based on correspondence; and acquisition using a function. The output information is not limited to information indicating whether or not a user has overpronation in his or her walking posture, and may be, for example, information indicating, in a stepwise manner, the degree at which a user can be said to have overpronation, or may be a score.

However, the output information is not limited to this, and in this embodiment, it may be determination information such as scores of individual findings or information indicating whether or not each finding is matched, or those containing such information. For example, the output information indicating whether or not the findings are matched may be acquired according to a result of a comparison between a score and a predetermined threshold value. The output information may be information generated from determination information such as a score or those containing such information, and may be, for example, a value calculated using respective scores of findings (e.g., a total value, etc.), information showing respective scores of findings in the form of a graph, information such as an image and a comment selected based on a predetermined correspondence according to respective scores of findings, a radar chart generated using respective scores of findings, or the like.

Furthermore, the output information may be those containing a suggestion for improving the kinesiological state of the body of a user. Such output information may be acquired, for example, as follows. For example, if the determination information indicates that a user has a strong tendency to overpronate in his or her walking posture, the output information acquiring unit 151 may acquire, as the output information, improvement suggestion information that introduces an approaches and the like that are considered effective for improving overpronation. The output information acquiring unit 151 may be configured, for example, to acquire output information using a correspondence table or the like in which correspondence between determination information and improvement suggestion information that is to be acquired as the output information is defined in advance, but the acquisition method is not limited to this. In this embodiment, specific examples of the improvement suggestion information include information on performing a predetermined exercise, information on matters that a user has to take into consideration, and the like, but there is no limitation to these. The suggestion for improving the kinesiological state of the body of a user may also be said to be, for example, a suggestion for improving a walking posture of the user. The suggestion for improving a walking posture may be, for example, items for improvement of the posture during walking or the like, or may be items for improvement that will have an effect on the posture during walking or the like. The items for improvement may be those regarding a condition during walking or those regarding a condition not during walking. The items for improvement regarding a condition during walking may be, for example, items for improvement regarding the force exerted on the toes during walking, items for improvement regarding the way the toes come into contact with the ground, or the like. The items for improvement regarding a condition not during walking may be, for example, items for improvement regarding a standing posture, items for improvement regarding awareness of the position in which weight is actively applied to the soles of the feet, or the like.

Furthermore, the output information may be those containing information on a product recommend for a user to ingest. For example, if the determination information indicates that a kinesiological state of the body of the user shows a predetermined condition, the output information acquiring unit 151 may be configured to acquire product information corresponding to the condition, as the output information. The product information is, for example, a product name, a product number, a product image, information such as a location where the product can be purchased, information such as a coupon code that can be redeemed for the product, or the like, but there is no limitation to these. The output information acquiring unit 151 may be configured, for example, to acquire output information using a correspondence table or the like in which correspondence between determination information and product information that is to be acquired as the output information is defined in advance, but the acquisition method is not limited to this.

The output information acquiring unit 151 may be configured to acquire output information, based on determination information acquired for a user this time and determination information acquired for the same user in the past. For example, the output information acquiring unit 151 may be configured to compare determination information acquired for a user and determination information acquired for the same user in the past, and acquire output information based on a result of the comparison. In such a case, for example, if it can be determined that the findings indicate that the condition is better this time than in the past based on the determination information acquired in the past and the determination information acquired this time, output information containing information to that effect may be acquired. For example, if a score or the like of a predetermined finding is acquired as determination information, information indicating whether or not the user's walking posture or the like is better this time than before can be acquired as the output information according to a result of a comparison between the past score and the current score. If such output information is used, it is possible to easily make the user aware of whether or not his or her condition is improving. If he output information contains product information on a product recommended for a user to ingest, the output information acquiring unit 151 may be configured to acquire product information, based on determination information acquired for a user and determination information acquired for the same user in the past. For example, the output information acquiring unit 151 may be configured to acquire product information, in the case in which the result of the comparison between the current determination information and the past determination information satisfies a predetermined acquisition condition.

The output unit 161 outputs the output information acquired by the output information acquiring unit 151. In this embodiment, the output unit 161 is configured to perform output by transmitting the output information to the information acquiring apparatuses 600 and other external apparatuses. However, there is no limitation to this, and the output unit 161 may be configured to output the output information by displaying the output information as characters or images on a display screen included in the information processing apparatus 100. If the output information is output in a user-recognizable manner, it is possible for the user, for example, to see the output information that is a score and know the score for each finding, or to see the output information indicating whether or not the user has overpronation in his or her walking posture and be aware of whether not the user has overpronation.

The transmitting unit 170 transmits information to other apparatuses constituting the information processing system 1 via a network. For example, the transmitting unit 170 transmits information to the information acquiring apparatuses 600. In other words, for example, the transmitting unit 170 outputs information to the information acquiring apparatuses 600.

In this embodiment, the information processing apparatus 100 may be configured to be able to generate learning information. Generation of learning information may be performed, for example, as follows.

Generation of learning information may be performed, for example, by the processing unit 140. The processing unit 140 generates learning information, for example, using a machine learning method. The machine learning method can be used, for example, as follows. That is to say, a classifier in which learning input information containing sole information is taken as input and determination information is taken as an output value is configured using a machine learning method. For example, two or more pairs of learning input information and output values (which may be referred to as training data) are prepared in advance, the two or more pairs of information are given to a module for configuring a classifier of machine learning to configure a classifier, and the configured classifier is accumulated in the learning information storage unit 111 as the learning information. There is no limitation on the machine learning method, and examples thereof include deep learning such as convolutional neural networks (CNN), random forests, SVM, and the like. For example, functions in various machine learning frameworks and various existing libraries, such as fastText, tinySVM, random forest, and TensorFlow, can be used for the machine learning. The combination of learning input information and output values may be prepared in advance. It is also possible that learning information is regenerated at a predetermined time using training data containing sole information and output value newly acquired in the information processing apparatus 100.

Next, the configuration of the information acquiring apparatuses 600 will be described.

As shown in the drawing, each information acquiring apparatus 600 includes a terminal storage unit 610, a terminal receiving unit 620, a terminal accepting unit 630, a terminal processing unit 640, a terminal output unit 660, a terminal transmitting unit 670, and a detecting unit 680. The information acquiring apparatus 600 is used, for example, to acquire sole information of a user who is an examinee.

Sole information acquired using the detecting unit 680 is accumulated in the terminal storage unit 610. The sole information is transmitted by the terminal transmitting unit 670 to the information processing apparatus 100, but, when the transmission is completed, it may be deleted from the terminal storage unit 610, be retained as it is until a predetermined period of time elapses, or be retained permanently until a deletion operation is performed by a user. User information of a user who is an examinee or the like may be further accumulated in the terminal storage unit 610.

The terminal receiving unit 620 receives information transmitted from the information processing apparatus 100 or other apparatuses, via a network. The terminal receiving unit 620 accumulates the received information, for example, in the terminal storage unit 610 so that it can be acquired by the terminal processing unit 640 and the like.

The terminal accepting unit 630 accepts various input operations to the information acquiring apparatus 600, which are performed by a user who uses the information acquiring apparatus 600. The operations are performed, for example, using an input apparatus (not shown), but there is no limitation to this.

The terminal processing unit 640 performs various types information processing operations using the units of the information acquiring apparatus 600.

The terminal output unit 660 has, for example, a display device or the like. The terminal output unit 660 outputs information by, for example, displaying the information on the display device. The method of outputting information is not limited to this, and may also be performed by outputting voice or other sounds from a speaker or the like.

The terminal transmitting unit 670 transmits, for example, information acquired by the terminal processing unit 640 or the like, via a network.

The detecting unit 680 is configured to be able to acquire sole information. In this embodiment, the detecting unit 680 is, for example, an image capturing device such as a camera. In this embodiment, the detecting unit 680 is configured to be able to capture an image of a sole of a user who is an examinee in a ground contacting state. For example, it can capture an image of a user's sole from below a transparent glass plate in a state where the user is barefoot on the glass plate. The image captured in this manner by the detecting unit 680 is accumulated in the terminal storage unit 610 as the sole information. The detecting unit 680 may acquire an image by capturing an image of a predetermined area or by performing scanning with a line sensor.

The storage unit 110 and the terminal storage unit 610 described above are preferably non-volatile recording media, but they may alternately be realized by volatile recording media. Information or the like acquired in each apparatus is stored in that apparatus, but the procedure in which information or the like is stored is not limited to this. For example, information or the like may be stored via a recording medium, information or the like transmitted via a communication line or the like may be stored, or information or the like input via an input device may be stored.

Furthermore, the processing unit 140 and the terminal processing unit 640 described above may be typically realized by MPUs, memories, or the like. Typically, the processing procedure of the processing unit 140 and the terminal processing unit 640 is realized by software, and the software is stored in a recording medium such as a ROM. Note that the processing procedure may be realized by hardware (dedicated circuits).

Furthermore, the information that can be accepted by the accepting unit 130 or the terminal accepting unit 630 may be input by any input part such as a numeric keypad, a keyboard, a mouse, or a menu screen. The accepting unit 130 and the terminal accepting unit 630 may be realized by device drivers for an input part such as a numeric keypad or a keyboard, control software for a menu screen, or the like.

Furthermore, the receiving unit 120 and the terminal receiving unit 620 are typically realized by wireless or wired communication parts, but they may also be realized by broadcast receiving parts.

Furthermore, the transmitting unit 170 and the terminal transmitting unit 670 are typically realized by wireless or wired communication parts, but they may also be realized by broadcasting parts.

The information processing apparatus 100 may be constituted by a single server, multiple servers that operate in conjunction with each other, or a computer or other device built into other equipment. The servers may be so-called cloud servers, ASP servers, or the like, and it will be appreciated that there is no limitation on the type thereof.

Next, an example of an operation of the information processing apparatus 100, which is performed when a user uses the information processing system 1 according to this embodiment, will be described. In this embodiment, the user can use the information processing system 1, for example, by accessing the information processing apparatus 100 or receiving information transmitted from the information processing apparatus 100 via the information acquiring apparatus 600 or other external apparatuses.

In this embodiment, the information processing system 1 is typically used, for example, as follows. That is to say, a user who is an operator acquires sole information of a user who is an examinee (who may be the same as the operator), using the information acquiring apparatus 600. Accordingly, the sole information is transmitted from the information acquiring apparatus 600 to the information processing apparatus 100, and the output information is acquired in the information processing apparatus 100. The information processing apparatus 100 transmits (outputs) the acquired output information to the information acquiring apparatus 600 or the like. This allows the user to check the output information. If such an operation of the information processing system 1 is performed, the information processing apparatus 100 performs various operations, for example, as follows. These operations are performed by the processing unit 140 executing control operations and the like while using the units.

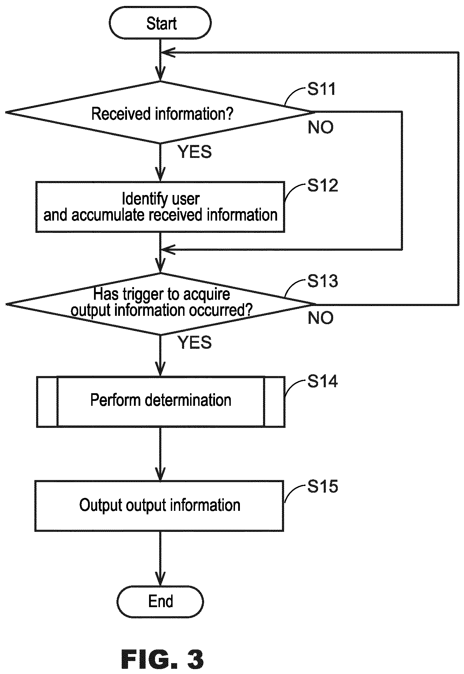

FIG. 3 is a flowchart showing an example of an operation of the information processing apparatus 100 in the embodiment.

(Step S11) The processing unit 140 determines whether or not the receiving unit 120 has received information transmitted from the information acquiring apparatus 600 or the like. If it is determined that the receiving unit 120 has received information, the procedure advances to step S12, or otherwise the procedure advances to step S13.

(Step S12) The processing unit 140 identifies a user based on the received information, and accumulates the received information in the storage unit 110 in association with a user identifier. For example, in the case in which the sole information is transmitted from the information acquiring apparatus 600 in association with a user identifier of a user who is an examinee, the processing unit 140 accumulates the received information in the sole information storage unit 117 in association with the user identifier. In the case in which user information is transmitted as well, the processing unit 140 accumulates it in the user information storage unit 115 in association with the user identifier.

(Step S13) The processing unit 140 determines whether or not a trigger to acquire output information has occurred. In other words, the processing unit 140 determines whether or not the condition for starting the acquisition of output information is satisfied. If it is determined that the trigger has occurred, the procedure advances to step S14. Otherwise, the procedure returns to step S11.

For example, the above-mentioned trigger may be, for example, that an instruction to acquire output information is given (predetermined information corresponding to the instruction is transmitted) from the user via the information acquiring apparatus 600 or the like, but the trigger is not limited to this. For example, the trigger may be that any of various conditions, such as the receipt of new sole information, is satisfied.

(Step S14) The processing unit 140 performs determination processing for acquiring output information. This processing will be described later. Accordingly, determination information and output information are acquired and accumulated in the storage unit 110.

(Step S15) The processing unit 140 causes the output unit 161 to output the output information to the target user. For example, the processing unit 140 causes the transmitting unit 170 to output the output information to the information acquiring apparatus 600 or the like that is used by the user who is an operator. This allows the information acquiring apparatus 600 or the like that received information to perform display or the like of the output information.

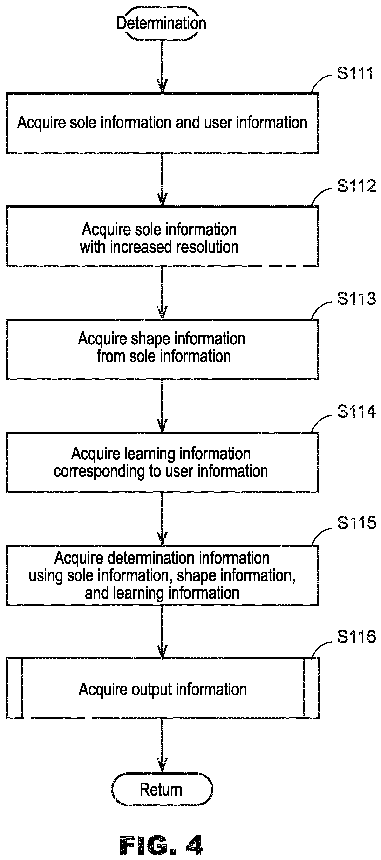

FIG. 4 is a flowchart showing an example of the determination processing by the information processing apparatus 100 in the embodiment.

(Step S111) The processing unit 140 acquires sole information, user information, and the like of a user who is an examinee, from the storage unit 110, based on a user identifier of that user.

(Step S112) The processing unit 140 performs processing for increasing the resolution of the sole information that is an image, thereby acquiring sole information whose resolution has been increased.

(Step S113) The processing unit 140 acquires shape information based on the sole information. The shape information is acquired in this case, for example, by performing an image recognition process or the like on the sole information.

(Step S114) The processing unit 140 acquires learning information corresponding to the user information, from the learning information storage unit 111. For example, the learning information that is acquired is information configured using training data for users who are in the same group as the user who is an examinee with respect to predetermined attributes such as age and weight.

(Step S115) The processing unit 140 applies the acquired sole information and shape information as information that is input, to the learning information, thereby acquiring determination information. For example, as the determination information, respective scores of the four findings are acquired as described above.

(Step S116) The processing unit 140 acquires output information using the determination information. The output information acquisition processing is performed, for example, as described below. Subsequently, the procedure returns to the upper-level processing.

The user information does not necessarily have to be acquired. The processing for increasing the resolution of sole information may not have to be performed. The shape information may not have to be acquired, and in that case, determination information may be acquired in step S115 using the sole information and the learning information.

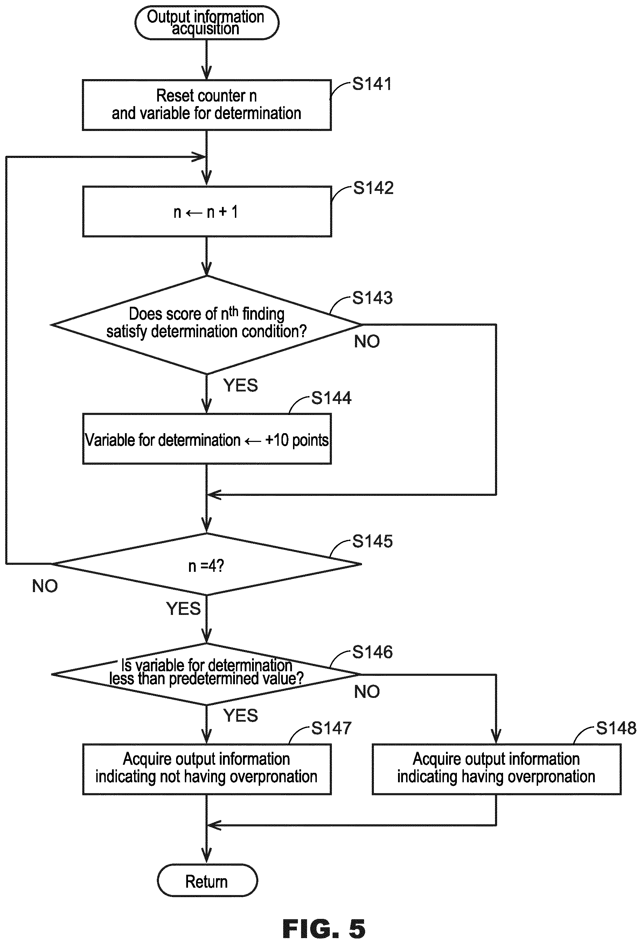

FIG. 5 is a flowchart showing an example of the output information acquisition processing by the information processing apparatus 100 in the embodiment.

(Step S141) The processing unit 140 resets a counter n and a variable for determination to their initial values (e.g., zero), respectively.

(Step S142) The processing unit 140 increments the counter n by 1.

(Step S143) The processing unit 140 determines whether or not a predetermined determination condition is satisfied by a score of an n^{th} finding in the determination information. If it is determined that the determination condition is satisfied, the procedure advances to step S144, or otherwise the procedure advances to step S145. The predetermined determination condition is, for example, that the score is greater than a predetermined value, but there is no limitation to this.

(Step S144) The processing unit 140 adds 10 points to the variable for determination.

(Step S145) The processing unit 140 determines whether or not the counter n is equal to 4. If n = 4, the procedure advances to step S146, or otherwise the procedure returns to step S142. That is to say, in this embodiment, step S146 and subsequent processing will be performed after whether or not the determination condition is satisfied is determined for each of the four findings.

(Step S146) The processing unit 140 determines whether or not the variable for determination is less than a predetermined value. If it is determined that the variable is less than a predetermined value, the procedure advances to step S147, or otherwise the procedure advances to step S148. The predetermined value can be set to 10, for example. That is to say, in this case, if the determination condition is satisfied by any of the respective scores of the findings, the procedure advances to step S147. The predetermined value may be set to other values, such as 20 or 30, for example.

(Step S147) The processing unit 140 acquires output information to the effect that the user does not have overpronation in his or her walking posture.

(Step S148) On the other hand, the processing unit 140 acquires output information to the effect that the user has overpronation in his or her walking posture.

If step S147 or step S148 is ended, the procedure returns to the upper-level processing. If the output information is output in this manner, the processing unit 140 outputs the output information. Subsequently, the series of processing is ended.

The output information acquisition processing may be processing for acquiring output information using second learning information as described above. In this case, the output information acquisition processing may be performed, for example, as follows.

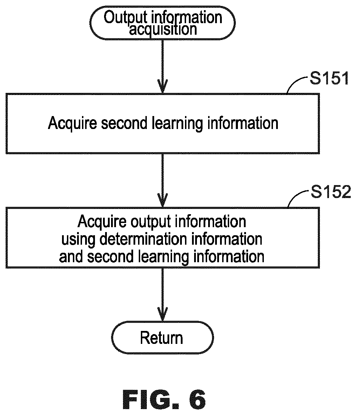

FIG. 6 is a flowchart showing a modified example of the output information acquisition processing by the information processing apparatus 100 in the embodiment.

(Step S151) The processing unit 140 acquires second learning information from the learning information storage unit 111.

(Step S152) The processing unit 140 applies the acquired determination information as information that is input, to the learning information, thereby acquiring output information. For example, as the output information, information indicating that the user has overpronation in his or her walking posture or information indicating that the user does not have overpronation in his or her walking posture is acquired. Subsequently, the procedure returns to the upper-level processing.

Furthermore, in the determination processing, for example, determination information indicating whether or not the user has overpronation may be acquired as the output information. In this case, the determination processing may be performed, for example, as follows.

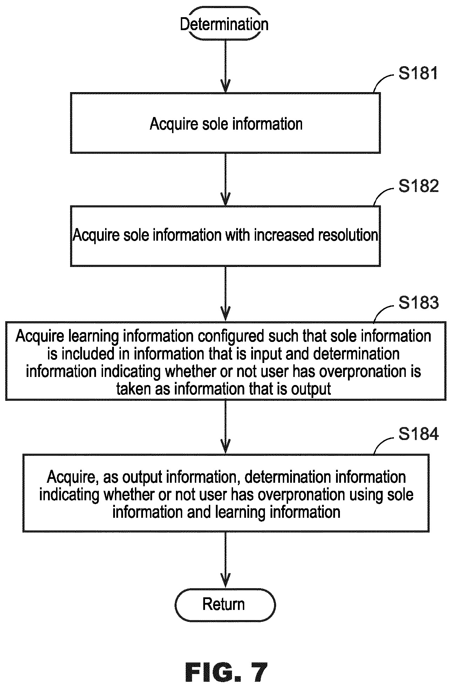

FIG. 7 is a flowchart showing a modified example of the determination processing by the information processing apparatus 100 in the embodiment.

(Step S181) The processing unit 140 acquires sole information of a user who is an examinee, from the storage unit 110, based on a user identifier of that user.

(Step S182) The processing unit 140 performs processing for increasing the resolution of the sole information that is an image, thereby acquiring sole information whose resolution has been increased.

(Step S183) The processing unit 140 acquires, from the learning information storage unit 111, learning information configured such that the sole information is included in information that is input and the determination information indicating whether or not the user has overpronation is taken as information that is output. The learning information may be configured and accumulated in advance in the learning information storage unit 111.

(Step S184) The processing unit 140 applies the acquired sole information as information that is input, to the learning information, thereby acquiring determination information. In this modified example, the processing unit 140 acquires determination information indicating whether or not the user has overpronation, as the output information. Subsequently, the procedure returns to the upper-level processing.

In the processing shown in FIG. 7, user information and shape information may be acquired and used in the same manner as that in FIG. 4.

In this embodiment, the output information acquired by the information processing apparatus 100 may be output to a user who is an operator or a user who is an examinee, for example, as follows. In the following specific example, it is assumed that the output information is transmitted to the information acquiring apparatus 600 that is a portable information terminal used by the user, and is displayed on its display screen. In the information acquiring apparatus 600, the output information is displayed, for example, by a predetermined application. When the user executes the predetermined application in the information acquiring apparatus 600, the information acquiring apparatus 600 communicates with the information processing apparatus 100, a screen is displayed by the terminal output unit 660 based on the control of the terminal processing unit 640 to realize the function of the predetermined application.

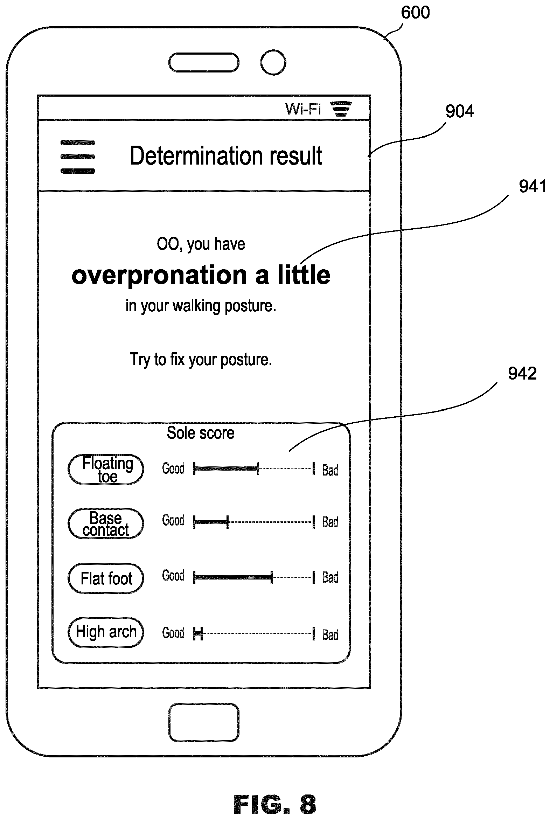

FIG. 8 is a diagram showing a specific example of a display screen of output information on the information acquiring apparatus 600 in the embodiment.

In the drawing, a result display screen 904 of a predetermined application is shown. The result display screen 904 includes, for example, a determination result display section 941 showing whether or not the user has overpronation and a score display section 942 showing a score for each finding acquired as determination information. The result display screen 904 allows the user to check the information indicating whether or not the user has overpronation and the determination information based on which the determination was made. The display mode of the output information is not limited to this. The information may not be displayed to the user who is an examinee, but may be displayed to a user who is different from the examinee, such as the user who is an operator.

As described above, the information processing apparatus 100 acquires determination information using learning information and sole information stored in the storage unit 110, and thus it is possible to easily specify information on a kinesiological state of the body of a user. In this embodiment, a captured image of a sole in a ground contacting state is used as the sole information, and thus it is possible to easily prepare the sole information.

In this embodiment, the information processing apparatus 100 can acquire output information regarding whether or not the user has overpronation and the like, using the determination information. Therefore, determinations that conventionally could only be made by judges with expert knowledge can be easily made. In addition, since there is no variation in determination by the judges, consistent determinations can be made.

The processing in this embodiment may be realized by software. The software may be distributed by software downloads or the like. Furthermore, the software may be distributed in a form where the software is stored in a recording medium such as an optical disk. The software that realizes the information processing apparatus 100 in this embodiment is the following sort of program. Specifically, this program is a program that is executed on a computer of the information processing apparatus 100, the program causing the computer to function as: a sole information acquiring unit that acquires sole information on a sole of a user; a determination information acquiring unit that acquires determination information on a kinesiological state of the user, using the sole information and learning information that is prepared in advance; an output information acquiring unit that acquires output information based on the determination information; and an output unit that outputs the output information.

### Description of Modified Example regarding Sole Information

In the foregoing embodiment, the following information may be used as the sole information.

For example, information on a sole that is not in contact with the ground may be used as the sole information. For example, a captured image of a sole that is not in contact with the ground may be used.

Furthermore, information indicating a three-dimensional shape of a user's sole may be used as the sole information. Such information may be, for example, point cloud data obtained by scanning a sole with a LiDAR scanner or the like. In this case, a three-dimensional shape of a sole that is not in contact with the ground may be used, or a three-dimensional shape of a sole in a ground contacting state may be used. The three-dimensional shape of a sole may be acquired, for example, by scanning a footprint obtained by molding a foot in a ground contacting state or a foot that is not in contact with the ground. For example, plaster, foam resin, silicon resin, clay, or the like may be used for the molding. If sole information that is such a three-dimensional shape is used, it is possible to obtain more accurate determination information.

Furthermore, information on the pressure distribution of a user's sole on a contacted ground surface may be used as the sole information. The information on the pressure distribution may be, for example, information containing values of pressure at the respective points on the contacted ground surface, or may be an image representing the pressure distribution. In the case in which an image representing the pressure distribution is used as the sole information, the determination information acquiring unit 149 may be configured to acquire determination information, using sole information obtained by performing processing for increasing the resolution of the sole information, as in the foregoing embodiment. Such information on the pressure distribution can be obtained immediately, and thus it is possible to easily acquire this information also as information dynamically representing a change in the ground contacting state of a user's sole during walking or the like. Accordingly, it is possible to easily obtain an accurate determination result regarding a posture during walking and the like.

If such information on the pressure distribution is used as the sole information, known pressure sensors can be used to acquire the sole information. That is to say, for example, a sheet-shaped pressure distribution sensor such as a pressure sensor mat that can measure a two-dimensional distribution of pressure on a surface can be used as the detecting unit 680 of the information acquiring apparatus 600. Also, for example, a so-called wearable pressure distribution sensor such as a pressure distribution sensor configured to be placeable on the insole portion of a shoe may be used as the detecting unit 680 of the information acquiring apparatus 600. If sole information can be acquired using such a wearable device, it is possible to monitor sole information at a higher frequency and acquire determination results, and to reduce the effort of measurement.

Furthermore, the sole information may be information read from a medium in which a ground contacting status of a user's sole is recorded. The medium may be, for example, pressure-sensitive paper or the like configured to discolor the area where pressure is applied. Also, the medium may be, for example, paper on which a user's footprint is transferred by ink or the like. Images or the like obtained by reading these media with a scanner or capturing them with a camera, which are the detecting unit 680 of the information acquiring apparatus 600, may be used as the sole information. If sole information can be acquired using a medium in which a ground contacting status of a user's sole is recorded in this manner, restrictions such as location and facilities for acquiring sole information can be eliminated, and thus it is possible to more easily acquire sole information.

In the foregoing embodiment, the sole information is, for example, information on the sole of one of the feet of a user, but there is no limitation to this. For example, information on the soles of both feet of a user may be used as the sole information. In this case, for example, determination information corresponding to each foot may be acquired based on information on the sole of that foot. For example, it is also possible that sole information corresponding to the right foot can be taken as input information and applied to the learning information to obtain determination information corresponding to the right foot, and sole information corresponding to the left foot can be taken as input information and applied to the learning information to obtain determination information corresponding to the left foot. In this case, different pieces of learning information may be used as that corresponding to the sole information corresponding to the right foot and that corresponding to the sole information corresponding to the left foot. The sole information corresponding to one of the feet may be configured to be used as a mirror image of the other foot. It is also possible that both information on the sole of the right foot and information on the sole of the left foot are taken as input information and applied to the learning information to acquire determination information. If determination information corresponding to the right foot and determination information corresponding to the left foot are obtained, these pieces of information may be used to acquire output information on the right foot and output information on the left foot. It is also possible that output information without differentiation between the left and the right is acquired. If sole information on the soles of both feet is used, it is possible to acquire more accurate determination information and output information.

### Description of Modified Example regarding Output Information

In the foregoing embodiment, the following information may be contained in the output information. For example, the following information may be contained in the output information in addition to the determination result regarding a walking posture of the user as described above, or the following information may be contained in the output information instead of the determination result regarding a walking posture of the user as described above.

That is to say, the output information may contain, for example, information on a symptom that may be currently occurring in a user (hereinafter, referred to as symptom information). The output information may contain information on a risk of a symptom that occurs in a user in the future (hereinafter, referred to as risk information). In other words, the output information acquiring unit 151 may be configured to acquire symptom information as the output information based on the determination information, or to acquire risk information as the output information based on the determination information. The output information acquiring unit 151 may be configured to acquire symptom information but not to acquire risk information, may be configured to acquire risk information without acquiring symptom information, or may be configured to acquire both symptom information and risk information as the output information.

The symptom in this case is, for example, sensations and conditions that are perceived by a user himself or herself, that is, those referred to as subjective symptoms, but there is no limitation to this. For example, the symptom may be conditions of a user that can also be perceived by someone other than the user, that is, objective symptoms. The symptom may be findings regarding a kinesiological state of a user, excluding those contained as the determination information.

The output information may contain a suggestion for improving the kinesiological state of the body of the user, together with such symptom information or risk information. That is to say, the output information may contain the suggestion for improving a walking posture as described above.

The output information may contain two or more pieces of symptom information, or may contain two or more pieces of risk information. If the output information contains two or more pieces of risk information, the output unit 161 may be configured to output the two or more pieces of risk information, in an order corresponding to when the respective symptoms are expected to occur. For example, among the two or more pieces of risk information, risk information on a symptom that is expected to occur earlier may be output earlier than the other pieces of risk information. For example, among the two or more pieces of risk information, risk information on a symptom that is expected to occur earlier may be output later than the other pieces of risk information. Outputting a piece of information earlier than the other pieces of information may be, for example, displaying it earlier in time or the like, or may be displaying it higher in the case in which the two or more pieces of information are displayed as a list. That is to say, the order in which the information is output in this case may mean a chronological order or a positional order. The output unit 161 may be configured to output two or more pieces of risk information contained in the output information, in such an order based on correspondence information in which risk information that can be taken as output information is associated in advance with ranking information on when the symptom corresponding to that risk information is expected to occur. The correspondence information may be stored in advance in the storage unit 110, for example, but there is no limitation to this. The ranking information in this case may be information indicating the order of output determined in advance according to when each symptom is expected to occur. The method for determining the order of output of risk information is not limited to this.

For example, if the determination information, the symptom information, the risk information, and the improvement suggestion are contained in the output information, the output unit 161 preferably outputs the output information such that these pieces of information are recognized by the user in the following order. The order in this case may mean a chronological order or a positional order. The order of output is not necessarily limited to this. Some of these pieces of information may not be contained in the output information.

That is to say, for example, the output unit 161 first performs output of determination information. For example, information indicating whether or not each finding is matched may be displayed for each finding. In this embodiment, for example, information indicating whether or not each of findings such as floating toe, base contact, flat foot, and high arch is matched is displayed. In this manner, each finding is matched.

Next, the output unit 161 performs output of symptom information. Examples of the symptom information that may be output include subjective symptoms that are relatively likely to be perceived by a user himself or herself, which may be currently caused by the user's walking posture, way of walking, or the like. For example, a subjective symptom that may match the user, from among the symptoms such as the calves being swollen, the legs being likely to be tired, the front thighs being likely to be tight, the hips being likely to spread to the side, the posture being likely to lean forward, or the like, may be output. If information on symptoms that are likely to be perceived by a user is output in this manner, it is possible to effectively make the user aware of the possibility of issues with his or her walking posture or way of walking. If information on pain that is familiar to the user is contained in the output information, it is possible to effectively make the user aware of the possibility of issues with his or her walking posture or way of walking.

Next, the output unit 161 performs output of risk information. Examples of the risk information that may be output include user's subjective symptoms or objective symptoms that are considered to have a certain possibility of occurring in the future depending on the user's walking posture, way of walking, or the like. Examples of the risk information include a tendency to have a foot corn, stiff ankles, the muscle built up inside the knees, hammer toes, bunion or bunionette, chronic pain in the knees, chronic pain in the hip joint, an increased risk of falling over, and the like. If information on the risk of symptoms that occurs in a user in the future is output in this manner, it is possible to effectively make the user aware of the possibility of issues with his or her walking posture or way of walking. Furthermore, if the future risks are presented, it is possible to effectively make the user aware of the need to take improvement actions to prevent the occurrence of symptoms. If the risk information is output together with the symptom information, it is possible to more effectively motivate the user to take the improvement actions. Since the user can be made aware that there is a risk of a symptom that may not have occurred yet, following the symptom information that the user may be currently feeling, it is possible to more strongly motivate the user to take improvement actions.

Subsequently, the output unit 161 performs output of improvement suggestions. The improvement suggestions may be, for example, items regarding the posture, items regarding the walking, or items regarding both. Examples of the items regarding the posture include to try to correct the standing posture, to try to stand on the outer sides in the feet, to align the direction of the knees and the toes, and the like. Examples of the items regarding the walking include to walk while relaxing the strength in the fingers, to walk with the thighs up without kicking the ground, and the like. If improvement suggestions are output in this manner, it is possible to make the user aware of what he or she has to do in order to improve the issues regarding his or her walking posture or way of walking. Since the contents of improvement actions can be specifically output and suggested to the user, the user can easily take improvement actions. If the improvement suggestions are output together with the symptom information or the risk information, it is possible to motivate the user to take improvement actions and provide guidance on the content thereof, and thus it is possible to easily and reliably have the user take improvement actions. If the improvement suggestions are output as well as the symptom information or the risk information, the user can be more strongly motivated to take improvement actions and also provided with guidance on specific content thereof, and thus it is possible to more reliably have the user take improvement actions.

The improvement suggestions may be output in such a way that the user can recognize specific items of improvement suggestions by illustrations or images, for example. It is also possible that the user is provided with guidance on items of improvement suggestions by video or the like.

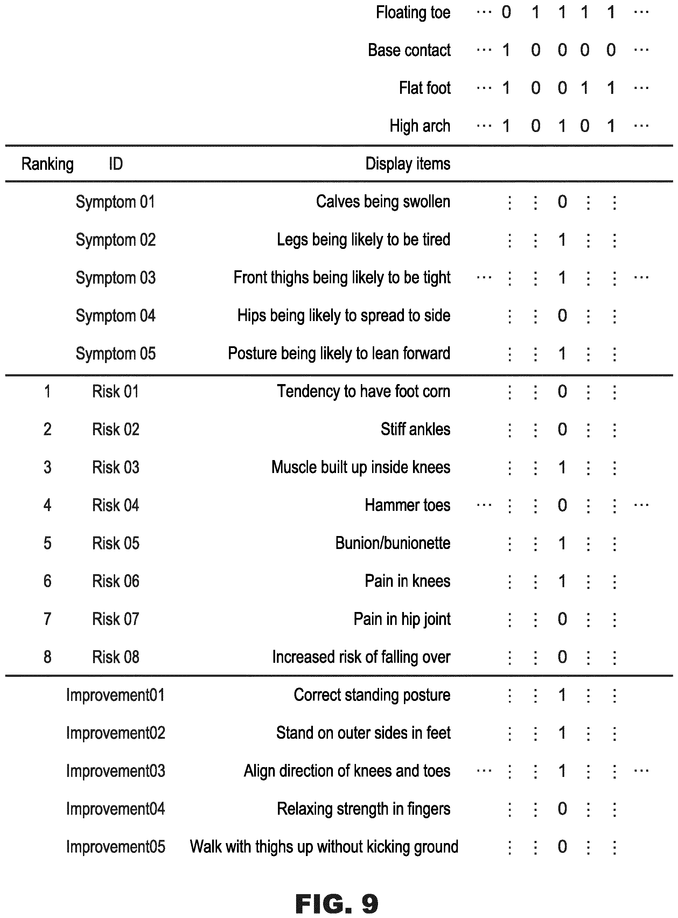

FIG. 9 is a diagram showing an example of information indicating the correspondence between determination information and output information or the like according to a modified example of this embodiment.

The processing according to the above-described modified examples can be realized using, for example, using correspondence information indicating the correspondence between determination information and output information or the like as shown in the drawing. In the example shown in this case, the determination information is a combination of information indicating whether each of the findings of floating toe, base contact, flat foot, and high arch is matched ("1") or not matched ("0"). The output information or the like contains two or more items of symptom information, two or more items of risk information, and two or more items of improvement suggestions. Each item is associated with information indicating whether each piece of determination information matches ("1") or does not match ("0") the output information.

The output information acquiring unit 151 can acquire, from the determination information, symptom information, risk information, improvement suggestions, and the like corresponding to the determination information, as the output information, based on such correspondence information. For example, in the example shown in the drawing, if the determination information is "1001" (matching the findings of floating toe and high arch), the following information will be contained in the output information. That is to say, the symptom information includes those related to the symptoms of the legs being likely to be tired, the front thighs being likely to be tight, and the posture being likely to lean forward. The risk information includes information indicating the risks related to the symptoms of the muscle built up inside the knees, bunion or bunionette, and pain in the hip joint. The improvement suggestions include the items to correct the standing posture, to stand on the outer sides in the feet, and to align the direction of the knees and the toes.

The ranking information is set for each item of risk information. The output unit 161 can output the risk information contained in the output information based on the ranking information. That is to say, for example, if the determination information is "1001" as mentioned above, the risk information will be output in the order of the muscle built up inside the knees, bunion or bunionette, and then pain in the hip joint, based on the ranking information corresponding to the respective pieces of information.

Such correspondence information is merely an example, and there is no limitation to this. The output information acquiring unit 151 may acquire symptom information, risk information, improvement suggestions, and the like, based on the determination information according to other methods. For example, it is also possible that these pieces of information are acquired based on a predetermined rule according to a determination result as to whether or not each of the multiple branching conditions is matched.

Such acquisition of output information according to this modified example may be performed, for example, as follows. In this case, it is assumed that information indicating whether or not each of the above-described four findings is matched is acquired as the determination information.

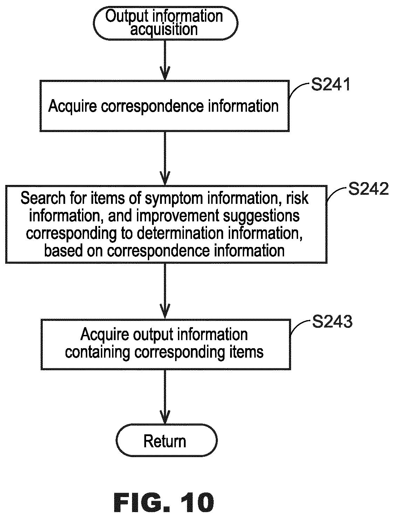

FIG. 10 is a flowchart showing an example of the output information acquisition processing by the information processing apparatus 100 according to a modified example of this embodiment.

The processing shown in the drawing is, for example, performed as step S116 of the determination processing in the foregoing embodiment. In step S115 of the determination processing, determination information indicating whether or not each of the four findings is matched is acquired.

(Step S241) The processing unit 140 acquires correspondence information indicating the correspondence between determination information and output information or the like. For example, correspondence information stored in advance in the storage unit 110 is acquired, but there is no limitation to this, and, for example, it is also possible that the information is acquired from an external apparatus or the like.

(Step S242) The processing unit 140 searches the correspondence information for the items of symptom information, risk information, and improvement suggestions corresponding to the determination information. Accordingly, the items corresponding to the determination information are acquired.

(Step S243) The processing unit 140 acquires output information containing the corresponding items. Subsequently, the procedure returns to the upper-level processing.

If output information is acquired in this manner, the output information is output later by the output unit 161.

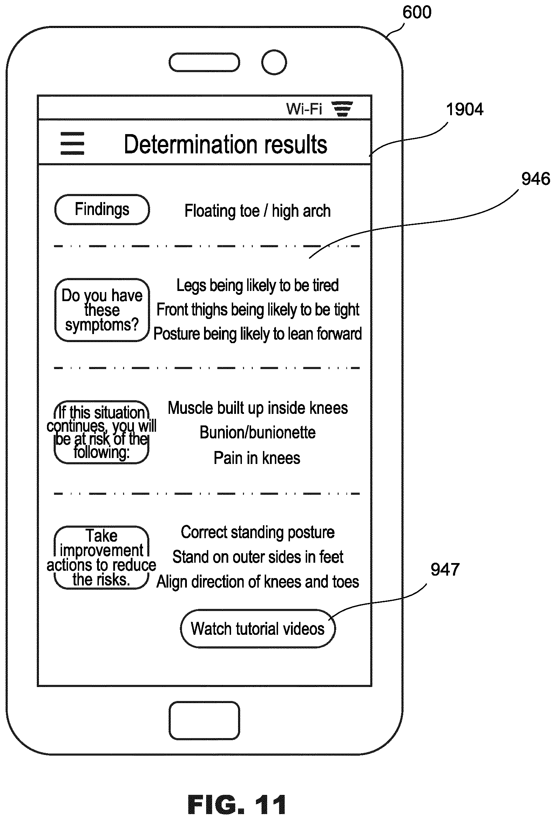

FIG. 11 is a diagram showing a specific example of a display screen of output information of the information acquiring apparatus 600 according to a modified example of this embodiment.

In the drawing, a result display screen 1904 of a predetermined application in which output information containing determination information, symptom information, risk information, improvement suggestions, and the like is output as the output information is shown. The result display screen 1904 includes an output information display section 946 containing the output information. Furthermore, a display button 947 for displaying the specific content of improvement suggestions is included. When the display button 947 is pressed, guidance information on the improvement suggestions such as how to practice specific improvement actions is displayed. Such guidance information may be included in the result display screen 1904. A determination result display section showing whether or not the user has overpronation may be included in the above-described result display screen 904. The user may check each of the symptom information, the risk information, and the improvement suggestions, on the result display screen 1904. The manner in which the output information is displayed is not limited to this. For example, one or more of the determination information, the symptom information, the risk information, and the improvement suggestions may be displayed by transitioning to another screen.

According to this modified example, symptom information, risk information, improvement suggestions, and the like are contained in the output information in this manner, and thus it is possible to effectively make the user aware of the possibility of issues with his or her walking posture or way of walking, the need to take improvement actions for the symptoms that have occurred and the risk of symptoms that occur in the future, and the like.

### Others

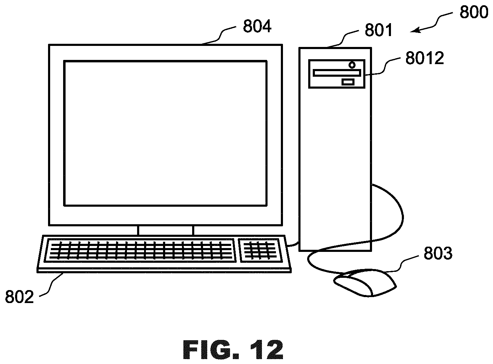

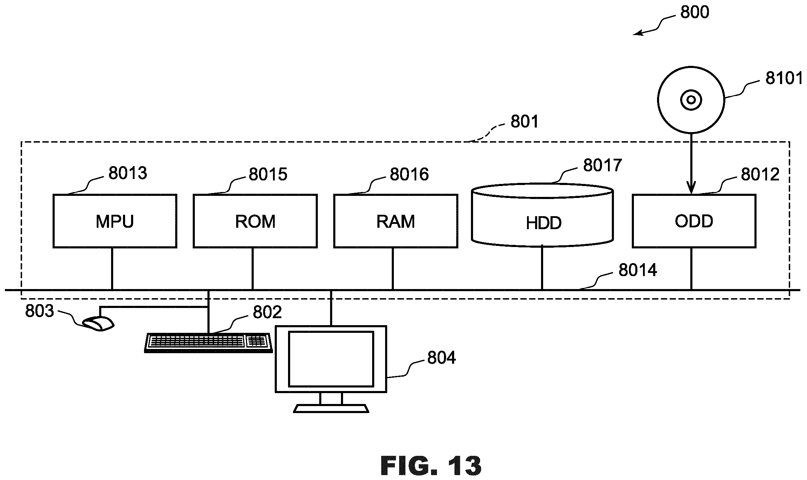

FIG. 12 is a schematic view of a computer system 800 in the foregoing embodiment. FIG. 13 is a block diagram of the computer system 800 in the foregoing embodiment.

These drawings shows a configuration example of a computer that executes the program described in this specification to realize the information processing apparatus and the like in the foregoing embodiment. The foregoing embodiment may be realized using computer hardware and a computer program executed thereon.

The computer system 800 includes a computer 801 including an optical disk drive, a keyboard 802, a mouse 803, and a monitor 804.

The computer 801 includes, in addition to the optical disk drive (ODD) 8012, an MPU 8013, a bus 8014 connected to the optical disk drive 8012 and the like, a ROM 8015 in which a program such as a boot up program is stored, a RAM 8016 that is connected to the MPU 8013 and is a memory in which a command of an application program is temporarily stored and a temporary storage area is provided, and a hard disk (HDD) 8017 in which programs such as application programs and system programs and data are stored. Although not shown, the computer 801 may further include a network card that provides connection to a LAN.

The program for causing the computer system 800 to execute the functions of the information processing apparatus and the like in the foregoing embodiment may be stored in an optical disk 8101 that is inserted into the optical disk drive 8012, and be transmitted to the hard disk 8017. Alternatively, the program may be transmitted via a network (not shown) to the computer 801 and stored in the hard disk 8017. At the time of execution, the program is loaded into the RAM 8016. The program may be loaded from the optical disk 8101, or directly from a network.

The program does not necessarily have to include, for example, an operating system (OS) or a third party program to cause the computer 801 to execute the functions of the information processing apparatus and the like in the foregoing embodiment. The program may only include a command portion to call an appropriate function (module) in a controlled mode and obtain desired results. The manner in which the computer system 800 operates is well known, and thus a detailed description thereof has been omitted.

It should be noted that, in the program, in a transmitting step of transmitting information, a receiving step of receiving information, or the like, processing that is performed by hardware, for example, processing performed by a modem or an interface card in the transmitting step (processing that can be performed only by hardware) is not included.

Furthermore, the computer that executes the program may be a single computer, or may be multiple computers. That is to say, centralized processing may be performed, or distributed processing may be performed.

Furthermore, in the foregoing embodiment, two or more constituent elements in one apparatus may be physically realized by one medium.

In the foregoing embodiment, each process (function) may be realized as centralized processing using a single apparatus (system), or may be realized as distributed processing using multiple apparatuses (in this case, the entire system constituted by multiple apparatuses that perform distributed processing may be regarded as one "apparatus").

Furthermore, in the foregoing embodiment, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiment, information related to the processing that is performed by each constituent element, for example, information that is to be accepted, acquired, selected, generated, transmitted, or received by each constituent element, information such as a threshold value, a numerical expression, or an address used by each constituent element in the processing and the like may be retained in an unshown recording medium temporarily or for a long period of time even if not specified in the description above. Furthermore, the information may be accumulated in the unshown recording medium by each constituent element or by an unshown accumulating unit. Furthermore, the information may be read from the unshown recording medium by each constituent element or by an unshown reading unit.

Furthermore, in the foregoing embodiment, if information used by each constituent element or the like, for example, information such as a threshold value, an address, or various setting values used by each constituent element in the processing may be changed by a user, the user may be or may not be allowed to change such information as appropriate even if not specified in the description above. If the user is allowed to change such information, the change may be realized by, for example, an unshown accepting unit that accepts a change instruction from the user and an unshown changing unit that changes information according to the change instruction. The unshown accepting unit may accept the change instruction, for example, by accepting information from an input device, by receiving information transmitted via a communication line, or by accepting information read from a predetermined recording medium.

The present invention is not limited to the embodiment and modified examples thereof set forth herein. Various modifications are possible within the scope of the present invention.

Some of the constituent elements or the functions may be omitted in the foregoing embodiment and modified examples thereof. Specific examples of the foregoing embodiment and modified examples thereof may be combined as appropriate.

The information processing apparatus according to the foregoing embodiment and modified examples thereof may also be said to be configured as follows. That is to say, this information processing apparatus is an information processing apparatus including:
a sole information acquiring unit that acquires sole information on a sole of a user;
a diagnostic information acquiring unit that acquires diagnostic information on a kinesiological state of the body of a user, using the sole information and learning information that is prepared in advance;
an output information acquiring unit that acquires output information based on the diagnostic information; and
an output unit that outputs the output information.

### Industrial Applicability

As described above, the information processing apparatus according to the present invention has an effect of making it possible to easily specify information on a kinesiological state of the body of a user, thus rendering this apparatus useful as an information processing apparatus and the like.

### List of Reference Numerals

- 1: Information processing system
- 100: Information processing apparatus
- 110: Storage unit
- 111: Learning information storage unit
- 115: User information storage unit
- 117: Sole information storage unit
- 120: Receiving unit
- 130: Accepting unit
- 140: Processing unit
- 141: Sole information acquiring unit
- 145: User information acquiring unit
- 149: Determination information acquiring unit
- 151: Output information acquiring unit
- 161: Output unit
- 170: Transmitting unit
- 600: Information acquiring apparatus
- 610: Terminal storage unit
- 620: Terminal receiving unit
- 630: Terminal accepting unit

- 640: Terminal processing unit
- 660: Terminal output unit
- 670: Terminal transmitting unit
- 680: Detecting unit

## Claims

1. An information processing apparatus comprising:
a sole information acquiring unit that acquires sole information on a sole of a user;
a determination information acquiring unit that acquires determination information on a kinesiological state of the body of the user, using the sole information and learning information that is prepared in advance; and
an output unit that outputs output information that is based on the determination information.

2. The information processing apparatus according to claim 1, wherein the sole information contains information on a ground contacting state in which the user's sole is in contact with a ground surface.

3. The information processing apparatus according to claim 2, wherein the sole information contains information indicating the ground contacting state of the user's sole in chronological order.

4. The information processing apparatus according to claim 1, wherein the sole information contains a captured image of the user's sole.

5. The information processing apparatus according to claim 1, wherein the sole information contains information on a pressure distribution of the user's sole that is in contact with a ground surface.

6. The information processing apparatus according to claim 1, wherein the sole information contains information indicating a three-dimensional shape of the user's sole.

7. The information processing apparatus according to claim 1, wherein the determination information acquiring unit is configured to perform processing for increasing resolution of the sole information that is an image, and use a processing result thereof and the learning information to acquire the determination information.

8. The information processing apparatus according to claim 1, wherein the sole information contains shape information on a shape of the user's sole.

9. The information processing apparatus according to claim 8, wherein the shape information contains at least one of a sole length and a sole width of the user.

10. The information processing apparatus according to claim 1, further comprising:
a user information acquiring unit that acquires information that is on the user and is different from the sole information, as user information,
wherein the determination information acquiring unit acquires the determination information using the user information.

11. The information processing apparatus according to claim 10, wherein the determination information acquiring unit acquires the determination information, using learning information corresponding to the user information among multiple pieces of learning information that are prepared in advance.

12. The information processing apparatus according to claim 1, wherein the determination information contains information on each of two or more findings regarding contact of a human sole with the ground.

13. The information processing apparatus according to claim 12, wherein the determination information contains respective scores of the two or more findings.

14. The information processing apparatus according to claim 12, wherein the determination information acquiring unit acquires information on each of the two or more findings, using learning information corresponding to the findings among multiple pieces of learning information that are prepared in advance.

15. The information processing apparatus according to claim 1, wherein the output information contains a determination result regarding a walking posture of the user.

16. The information processing apparatus according to claim 1, wherein the output information contains a suggestion for improving the kinesiological state of the body of the user.

17. The information processing apparatus according to claim 1, wherein the output information contains information on a product recommended for the user to ingest.

18. The information processing apparatus according to claim 1, wherein the output information contains information on a symptom that may be currently occurring in the user.

19. The information processing apparatus according to claim 1, wherein the output information contains information on a risk of a symptom that occurs in the user in the future.

20. The information processing apparatus according to claim 19,
wherein the output information contains two or more pieces of information on risks of symptoms that occur, and
the output unit outputs the two or more pieces of information on risks of symptoms that occur, in an order corresponding to when the respective symptoms are expected to occur.

21. The information processing apparatus according to claim 1, wherein the output information is information acquired based on determination information acquired for the user this time and determination information acquired for the same user in the past.

22. An information processing system comprising:
the information processing apparatus according to any one of claims 1 to 21; and
an information acquiring apparatus capable of communicating with the information processing apparatus,
wherein the information acquiring apparatus is configured to record information on a sole of a user and output a recorded result as sole information, and
the sole information acquiring unit is configured to acquire the sole information output by the information acquiring apparatus.

23. An information processing method realized using a sole information acquiring unit, a determination information acquiring unit, and an output unit, comprising:
a sole information acquiring step of the sole information acquiring unit acquiring sole information on a sole of a user;
a determination information acquiring step of the determination information acquiring unit acquiring determination information on a kinesiological state of the user, using the sole information and learning information that is prepared in advance; and
an output step of the output unit outputting output information that is based on the determination information.

24. A program for causing a computer to function as:
a sole information acquiring unit that acquires sole information on a sole of a user;
a determination information acquiring unit that acquires determination information on a kinesiological state of the user, using the sole information and learning information that is prepared in advance; and
an output unit that outputs output information that is based on the determination information.
